# EUROPÄISCHE PATENTSCHRIFT

(11) **EP 0 570 684 B1**
(45) Veröffentlichungstag und Bekanntmachung des Hinweises auf die Patenterteilung: **19.02.1997**
(21) Anmeldenummer: 93104862.3
(22) Anmeldetag: 24.03.1993
(51) Int. Cl.: G01N 1/24

(54) **Gasentnahmevorrichtung für ein Rauchgas-Analysegerät**
Gas sampling device for combustion gas analyser
Dispositif de prélèvement de gaz pour analyseur de fumée

(30) Priorität: 18.05.1992 DE 4216404
(43) Veröffentlichungstag der Anmeldung: 24.11.1993
(73) Patentinhaber: TESTOTERM FRITZSCHING GmbH & Co., D-79853 Lenzkirch (DE)
(72) Erfinder: Springmann, Thomas, W-7800 Freiburg (DE)
(74) Vertreter: Patentanwälte Westphal, Buchner, Mussgnug Neunert, Göhring

(56) Entgegenhaltungen:
- EP-A- 0 324 330
- DE-A- 1 949 081
- DE-A- 2 416 576
- US-A- 3 881 359
- US-A- 4 974 455
- PATENT ABSTRACTS OF JAPAN vol. 5, no. 69 (P-060)9. Mai 1981

## Beschreibung

Die Erfindung betrifft einen Gasentnahmeschlauch für ein Rauchgas-Analysegerät zum mobilen Einsatz gemäß Oberbegriff des Anspruchs 1.

Zur Analyse des Rauchgases von Feuerungsanlagen wird das Rauchgas mittels einer Sonde angesaugt und einem Analysegerät zugeführt. Für die Gasanalyse muß das Rauchgas aufbereitet werden, d.h. insbesondere Rauch- und Schmutzpartikel sowie Feuchtigkeit müssen aus dem Rauchgas entfernt werden.

Das Rauchgas wird von der Sonde über einen Schlauch zu dem Analysegerät geleitet. Schlägt sich Kondensat in dem Schlauch nieder, so kommt das Rauchgas in dem Schlauch über eine lange Verweildauer mit dem flüssigen Kondensat in Berührung. Dabei wird NO₂ und SO₂ teilweise in dem flüssigen Kondensat gebunden, so daß Meßfehler auftreten.

Um derartige Meßfehler zu vermeiden, ist es bekannt, einen beheizten Schlauch zu verwenden. In dem Schlauch wird das Rauchgas auf eine Temperatur oberhalb des Taupunktes erwärmt, so daß kein Kondensat abgeschieden wird. Die gesamte Kondensatabscheidung erfolgt in einer dem Analysegerät vorgeschaltete gekühlten Kondensatfalle. Dadurch wird bewirkt, daß das Kondensat schnell und in einem kurzen Bereich des Gasströmungsweges abgeschieden wird, so daß die Berührungsdauer des Gases mit dem flüssigen Kondensat begrenzt ist und NO₂ und SO₂ nur in einem geringen, das Meßergebnis unerheblich verfälschenden Ausmaß gebunden werden.

Um Schmutz- und Rußpartikel aus dem dem Analysegerät zugeführten Rauchgas zu entfernen, ist es bekannt, zwischen die Rauchgassonde und den beheizbaren Schlauch einen Filter einzusetzen. Nachteilig ist dabei, daß sich in diesem Filter bereits Kondensat niederschlagen kann. Dies führt zu der oben genannten Verfälschung des Meßergebnisses, da in dem Kondensat NO₂ und SO₂ teilweise gebunden wird. Außerdem führt die Feuchtigkeit des Kondensats in dem Filter zu einem schnelleren Zusetzen des Filters durch Schmutz- und Rauchpartikel.

Zur Umgehung dieses Problems ist bereits vorgeschlagen worden, den Filter mit einer elektrischen Heizeinrichtung zu versehen. Diese befindet sich im Inneren eines Gehäuses und ist beispielsweise gemäß der DE-OS 19 49 081 oder der EP-A-0 324 330 in Form einer Heizspirale um das Filterelement oder gemäß der US 3,881,359 neben dem Filterelement ausgeführt, welches sich ebenfalls im Gehäuse befindet. Hierdurch gelingt es, die Temperatur des Rauchgases oberhalb des Taupunktes zu halten. Der Nachteil bei einer derartigen Konfiguration besteht darin, daß der beheizbare Schlauch bzw. die beheizbare Meßgasleitung und der vorgeschaltete beheizbare Filter als separate Einheiten vorliegen und jeweils eine eigene Heizung aufweisen. Der erforderliche Aufwand ist hoch; insbesondere hinsichtlich der Abmessungen und des Gewichts ergeben sich Probleme, wenn die Analyse des Rauchgases nicht stationär sondern durch mobile Einheiten erfolgen soll.

Der Erfindung lag daher das Problem zugrunde, einen Gasentnahmeschlauch für ein Rauchgas-Analysegerät zum mobilen Einsatz zur Verfügung zu stellen, der die beschriebenen Nachteile nicht mehr aufweist. Insbesondere soll im Hinblick auf eine mobile Einsatzmöglichkeit ein Gasentnahmeschlauch mit geringem Gewicht und geringen Abmessungen realisiert werden.

Gelöst wird dieses Problem mit einem Gasentnahmeschlauch, der die Merkmale des Anspruchs 1 aufweist.

Vorteilhafte Ausführungsformen des Gasentnahmeschlauchs sind in den Unteransprüchen angegeben.

Der Erfindung liegt die Idee zugrunde, den Partikelfilter im Gasentnahmeschlauch zu integrieren. Gleichzeitig wird auf eine eigene, separate Heizung für den Partikelfilter dadurch verzichtet, daß der Partikelfilter von dem Heizmantel der Meßgasleitung temperaturgeregelt beheizt wird. Der konstruktive Aufwand reduziert sich durch diese Maßnahme erheblich, da nicht nur die ansonsten separate Heizung für den Partikelfilter vollständig entfällt, sondern auch keine weiteren Anschlüsse für die Stromversorgung vorzusehen sind. Auch vereinfacht sich die Bedienung erheblich, da eine separate Handhabung des Partikelfilters entfällt und damit Fehlbedienungen insoweit ausgeschlossen sind.

Die Erfindung wird nachstehend anhand der Figur näher erläutert. Sie zeigt schematisch den erfindungsgemäßen Meßgasschlauch in Schnittdarstellung.

Der Gasentnahmeschlauch 2 weist eine Meßgasleitung 21 auf, die von einer Heizwicklung 22 umgeben ist. Die Heizwicklung 22 ist vollständig von einer Isolierschicht 23 nach Art eines Schlauches umgeben. Die Heizwicklung 22 und die Isolierschicht 23 bilden den sogenannten Heizmantel. Die Versorgung der Heizwicklung 22 mit elektrischer Energie erfolgt durch eine Anschlußleitung 6, die über eine Kupplung 60 in hier nicht näher dargestellter Art und Weise mit der ebenfalls hier nicht dargestellten Stromquelle verbunden ist. Zum Schutz gegen mechanische Beschädigung ist eine Armierung 24 vorgesehen, die den Heizmantel nach Art eines Schlauches vollständig umschließt.

Der Partikelfilter 1 besitzt ein Filterelement 11. Er hat die Form eines Hohlzylinders und wird vom Rauchgas in radialer Richtung von außen nach innen durchströmt. Beim Durchtritt des Rauchgases durch das Filterelement 11 werden die Schmutz- und Staubpartikel zurückgehalten, das von den Staub- bzw. Schmutzpartikeln befreite Rauchgas tritt in die Meßgasleitung 21 ein und durchströmt diese. Der Partikelfilter ist koaxial und eingangsseitig im Gasentnahmeschlauch 2 integriert. Hierzu ist der Heizmantel in koaxialer Richtung über das Ende der Meßgasleitung 21 hinaus verlängert und umschließt den Partikelfilter. Er ist annähernd vollständig in axialer Erstreckung um den Partikelfilter 1 geführt, so daß das Rauchgas bereits unmittelbar nach dem Eintritt in den Bereich des Partikelfilters 1 von der Heizwirkung erfaßt wird, eine unerwünschte Abkühlung des Rauchgases wird dadurch vermieden.

Das zylinderförmige Filterelement 11 ist zentriert in einem koaxialen Filtergehäuse 10 gelagert. Das Filtergehäuse 10 ist ebenfalls zylinderförmig gestaltet. Seine Außenwand ist in Berührung mit der Heizwicklung 22. Der Innendurchmesser des Filtergehäuses 10 ist so gewählt, daß zwischen seiner Innenwand und dem im Filtergehäuse 10 zentriert gelagerten Filterelement 11 ein Zuströmbereich für das eintretende Rauchgas entsteht.

Zur Zentrierung des Filterelements 11 ist zuströmseitig ein Verschlußstopfen 12 für das Filtergehäuse 10 vorgesehen. Der Verschlußstopfen 12 ragt mit einem Zentrierzapfen 120 in das Innere des Filtergehäuses 10 hinein. Der Zentrierzapfen 120 greift in das Innere des zylinderförmigen Filterelements 11 ein. Abströmseitig wird das Filterelement 11 durch einen Absatzsteg 101 zentriert, der sich innenseitig im Filtergehäuse 10 befindet.

Der Verschlußstopfen 12 weist einen Konus 125 auf, der am trichterförmigen Abschluß 105 des Filtergehäuses 10 zur Anlage gebracht ist. Die Fixierung erfolgt über Verschraubungselemente 40, 41, die ein einfaches Auswechseln des Filterelements 11 ermöglichen. Eine Überwurfmutter 40 ist im Eingriff mit einer Spannhülse 41, welche sich über die Rückseite des trichterförmigen Abschlusses 105 am Filtergehäuse 10 abstützt. Die Überwurfmutter 40 wirkt auf eine Distanzhülse 42 ein, die im rückwärtigen Bereich des Konus 125 auf den Verschlußstopfen 12 drückt. Durch das Anziehen der Überwurfmutter 40 wird somit der Verschlußstopfen 12 über den Konus 125 mit dem Filtergehäuse 10 an dessen trichterförmigem Abschluß 105 verspannt. Am Filtergehäuse 10 und am Verschlußstopfen 12 ist jeweils ein Sicherungsring 104, 124 angebracht, die die Axialverschiebung der Verschraubungselemente 40, 41 in geöffnetem Zustand begrenzen.

Der Verschlußstopfen 12 weist weiterhin an seinem vorderen Ende einen Anschlußnippel 127 auf, an den in hier nicht dargestellter Art und Weise eine Gasentnahmesonde angeschlossen werden kann. Die Zuführung des Rauchgases zum Filterelement 11 wird durch Führungskanäle 128, 129 ermöglicht, die in Form von Bohrungen im Verschlußstopfen 12 angebracht sind. Das Rauchgas tritt somit axial in den Führungskanal 128 ein und wird durch den radial verlaufenden Führungskanal 129 in den Zwischenraum zwischen dem Filtergehäuse 10 und dem Filterelement 11 geleitet.

Zur Erhöhung der mechanischen Stabilität weist der Gasentnahmeschlauch 2 sowohl eingangsseitig als auch ausgangsseitig Abdeckhülsen 20, 30 auf. Eingangsseitig erstreckt sich die Abdeckhülse 20 in axialer Richtung im Bereich des Partikelfilters 1. Die Abdeckhülse 20 wird stirnseitig von einem Lagerring 201 verschlossen, der das Filtergehäuse 10 aufnimmt. Die Abdeckhülse 20 ist an ihrem anderen Ende über die Armierung 24 geführt, die im Bereich der Überlappung eine kleine Erhöhung in Form eines Wulstes 241 aufweist. Hierdurch wird eine Klemmwirkung zwischen der Abdeckhülse 20 und der Armierung 24 erzielt, die einerseits als Abdichtung und andererseits als Zugentlastung dient.

Abgangsseitig ist in gleicher Weise die Klemmhülse 30 angebracht, durch die die elektrische Anschlußleitung 6 für die Heizwicklung 22 hindurchgeführt ist. Die Abdeckhülse 30 besitzt ebenfalls einen Lagerring 301, der einen abgangsseitigen Anschlußnippel 302 aufnimmt.

Abgangsseitig weist der Gasentnahmeschlauch 2 ebenfalls einen Wulst 242 auf, der von der Abdeckhülse 30 klemmend umschlossen wird.

Die Wülste 241 und 242 ergeben sich dadurch, daß Klemmhülsen 211 und 212 über die Meßgasleitung 21 im Bereich des Übergangsnippels 102 bzw. des Anschlußnippels 302 geschoben sind und infolgedessen an dieser Stelle der Heizmantel einschließlich der Armierung 24 radial nach außen verdrängt werden.

## Patentansprüche

1. Gasentnahmeschlauch für ein Rauchgas-Analysegerät zum mobilen Einsatz, bestehend wenigstens aus einem Partikelfilter, der mit einer elektrischen Heizeinrichtung temperaturgeregelt heizbar ist und einer Meßgasleitung sowie einem Heizmantel, mit dem die Meßgasleitung temperaturgeregelt heizbar ist, dadurch gekennzeichnet, daß der Partikelfilter (1) im Gasentnahmeschlauch (2) integriert ist und gemeinsam mit der Meßgasleitung (21) mit dem Heizmantel (22,23) temperaturgeregelt heizbar ist.

2. Gasentnahmeschlauch nach Anspruch 1, dadurch gekennzeichnet, daß der Partikelfilter (1) koaxial und eingangsseitig im Gasentnahmeschlauch (2) integriert ist.

3. Gasentnahmeschlauch nach einem der vorstehenden Ansprüche, dadurch gekennzeichnet, daß der Heizmantel (22,23) den Partikelfilter (1) wenigstens annähernd vollständig in seiner koaxialen Erstreckung umschließt.

4. Gasentnahmeschlauch nach einem der vorstehenden Ansprüche, dadurch gekennzeichnet, daß der Partikelfilter (1) ein Filterelement (11) in Form eines Hohlzylinders aufweist, das zentriert in einem koaxialen Filtergehäuse (10) gelagert ist.

5. Gasentnahmeschlauch nach Anspruch 4, dadurch gekennzeichnet, daß das Filtergehäuse (10) zuströmseitig mit einem Verschlußstopfen (12) verschlossen ist, welcher Führungskanäle (128,129) für das Meßgas aufweist und mit einem Zentrierzapfen (120) zur Aufnahme des Filterelements (11) versehen ist.

6. Gasentnahmeschlauch nach Anspruch 5, dadurch gekennzeichnet, daß der Verschlußstopfen (12) über Verschraubungselemente (40,41) am Filtergehäuse (10) fixierbar ist.

7. Gasentnahmeschlauch nach einem der Ansprüche 4 bis 6, dadurch gekennzeichnet, daß das Filtergehäuse (10) abströmseitig innenseitig einen Absatzsteg (101) zur Zentrierung des Filterelements (11) aufweist.

8. Gasentnahmeschlauch nach einem der vorstehenden Ansprüche, dadurch gekennzeichnet, daß der Heizmantel (22,23) aus einer die Heizwicklung (22) schlauchförmig umgebenden Isolierschicht (23) besteht.

9. Gasentnahmeschlauch nach einem der vorstehenden Ansprüche, dadurch gekennzeichnet, daß er eingangsseitig eine Abdeckhülse (20) aufweist, die sich in axialer Richtung im Bereich des Partikelfilters (1) erstreckt.

10. Gasentnahmeschlauch nach einem der vorstehenden Ansprüche, dadurch gekennzeichnet, daß er abgangsseitig eine Abdeckhülse (30) aufweist, durch die eine elektrische Anschlußleitung (6) für die Heizwicklung (22) geführt ist.

11. Gasentnahmeschlauch nach einem der vorstehenden Ansprüche, dadurch gekennzeichnet, daß er eine Armierung (24) aufweist.

## Claims

1. Gas sampling tube for a combustion gas analyser for mobile use, comprising at least one particle filter which can be heated in a temperature-regulated manner using an electrical heating device, a test-gas line and also a heating jacket with which the test-gas line can be heated in a temperature-regulated manner, characterized in that the particle filter (1) is integrated in the gas sampling tube (2) and can be heated in a temperature-regulated manner together with the test-gas line (21) using the heating jacket (22, 23).

2. Gas sampling tube according to Claim 1, characterized in that the particle filter (1) is integrated in the gas sampling tube (2) coaxially and at the inlet end.

3. Gas sampling tube according to one of the preceding claims, characterized in that the heating jacket (22, 23) encloses the particle filter (1) at least approximately completely in its coaxial extension.

4. Gas sampling tube according to one of the preceding claims, characterized in that the particle filter (1) has a filter element (11) in the form of a hollow cylinder which is mounted in a centred manner in a coaxial filter housing (10).

5. Gas sampling tube according to Claim 4, characterized in that the filter housing (10) is sealed on the inflow side with a closure stopper (12) which has conduction channels (128, 129) for the test gas and is provided with a centring stud (120) for receiving the filter element (11).

6. Gas sampling tube according to Claim 5, characterized in that the closure stopper (12) can be fixed to the filter housing (10) by means of screw-joint elements (40, 41).

7. Gas sampling tube according to one of Claims 4 to 6, characterized in that the filter housing (10) has an internally stepped section (101) on the outflow side for centring the filter element (11).

8. Gas sampling tube according to one of the preceding claims, characterized in that the heating jacket (22, 23) comprises an insulating layer (23) surrounding the heater winding (22) in tube form.

9. Gas sampling tube according to one of the preceding claims, characterized in that it has on the inlet side a covering sleeve (20) which extends in the axial direction in the region of the particle filter (1).

10. Gas sampling tube according to one of the preceding claims, characterized in that it has on the outlet side a covering sleeve (30) through which an electrical connecting line (6) for the heater winding (22) is passed.

11. Gas sampling tube according to one of the preceding claims, characterized in that it has an armouring (24).

## Revendications

1. Tube de prélèvement de gaz destiné à un appareil d'analyse de gaz de fumée à cartouche mobile, comportant au moins un filtre à particules pouvant être chauffé électriquement avec régulation de température, une canalisation de mesure de gaz ainsi qu'une enveloppe chauffante permettant de réguler la température de la canalisation de mesure,
caractérisé en ce que
le filtre à particules (1) est intégré au tube de prélèvement du gaz (2) et peut être chauffé avec régulation de température, en même temps que la canalisation de mesure (21), par l'enveloppe chauffante (22, 23).

2. Tube de prélèvement selon la revendication 1,
caractérisé en ce que
le filtre à particules (1) est intégré au tube de prélèvement (2) coaxialement et du côté de l'entrée du gaz.

3. Tube de prélèvement selon une des revendications précédentes,
caractérisé en ce que
l'enveloppe chauffante (22, 23) entoure le filtre à particules (1) au moins sur presque toute sa longueur axiale.

4. Tube de prélèvement selon une des revendications précédentes,
caractérisé en ce que
le filtre à particules (1) comprend un élément filtrant (11) ayant la forme d'un cylindre creux, monté centré à l'intérieur du boîtier de filtre (10), coaxial à l'élément.

5. Tube de prélèvement selon la revendication 4,
caractérisé en ce que
le boîtier de filtre (10), du côté de l'entrée des gaz, est fermé par un bouchon obturateur (12) qui est percé de canaux (128, 129) conduisant le gaz à mesurer et qui porte un tourillon de centrage (120) destiné à recevoir l'élément filtrant (11).

6. Tube de prélèvement selon la revendication 5,
caractérisé en ce que
le bouchon obturateur (12) peut être fixé sur le boîtier de filtre (10), par des éléments filetés (40, 41).

7. Tube de prélèvement selon l'une des revendications 4 à 6,
caractérisé en ce que
le boîtier de filtre (10), vers sa sortie, présente sur sa paroi interne une bride en gradin (10) servant à centrer l'élément filtrant (11).

8. Tube de prélèvement selon l'une des revendications précédentes,
caractérisé en ce que
l'enveloppe chauffante (22, 23) est constituée d'une couche isolante (23) entourant, à la manière d'un tube, l'enroulement chauffant (22).

9. Tube de prélèvement selon une des revendications précédentes,
caractérisé en ce qu'
il comporte à l'entrée une douille de couverture (20) disposée, selon la direction axiale, au niveau du filtre à particules (1).

10. Tube de prélèvement selon une des revendications précédentes,
caractérisé en ce qu'
il comporte à la sortie une douille de couverture (30) traversée par un conducteur électrique (6) de raccordement de l'enroulement chauffant (22).

11. Tube de prélèvement selon une des revendications précédentes,
caractérisé en ce qu'
il comporte une armature (24).
